(19) Europäisches Patentamt
European Patent Office
Office européen des brevets

(11) Publication number: **0 040 400**
**B1**

(12) **EUROPEAN PATENT SPECIFICATION**

(45) Date of publication of patent specification: **16.05.84**

(51) Int. Cl.³: **C 07 C 43/23**, C 07 C 41/01, C 07 C 41/16

(21) Application number: **81103694.6**

(22) Date of filing: **13.05.81**

(54) **Process for preparing the mono-methallyl-ether of pyrocatechin.**

(30) Priority: **16.05.80 IT 2212980**

(43) Date of publication of application:
**25.11.81 Bulletin 81/47**

(45) Publication of the grant of the patent:
**16.05.84 Bulletin 84/20**

(84) Designated Contracting States:
**BE CH DE FR GB LI NL**

(56) References cited:
**DE-B-2 552 713**
**GB-A-2 006 211**
**GB-A-2 027 427**

**JOURNAL OF ORGANOMETALLLIC CHEMISTRY, Vol. 116, No. 1, 1976 Lausanne S. CABIDDU et al. "Cleavage of the Ethereal Bond. XI. The Reaction of Allylic Organomagnesium Halides with 1,3-Benzoxathiole and 1,3-Benzodioxole" pages 275 to 279**

(73) Proprietor: **Montedison S.p.A.**
**Patents & Licensing Dept. Foro Buonaparte, 31**
**P.O. Box 10528**
**I-20121 Milan (IT)**

(72) Inventor: **Campolmi, Stefano, Dr.**
**27, Via Andrea Costa**
**Novara (IT)**
Inventor: **Carletti, Vittorio**
**7, Largo Europa**
**Meda (Milan) (IT)**
Inventor: **Marchi, Marcello**
**11, Viale Allegra**
**Novara (IT)**

(74) Representative: **Schmied-Kowarzik, Volker, Dr. et al**
**Siegfriedstrasse 8**
**D-8000 München 40 (DE)**

**EP 0 040 400 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a process for preparing the mono-methallyl-ether of pyrocatechol of formula (I):

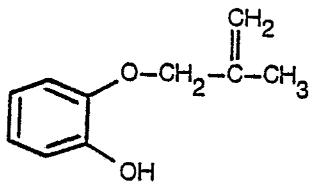

(I)

More precisely, the present invention relates to a process for the selective synthesis of the mono-methallyl-ether of pyrocatechin of formula (I) by etherification of pyrocatechol (II):

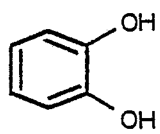

(II)

with a methallyl halide of formula (III):

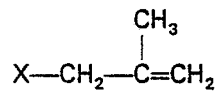

(III)

wherein X is Cl, Br, J and preferably chlorine, in a hydroxylated homogeneous dissolving medium in the presence of an organic or inorganic base.

The resulting compound (I) is an important intermediate for organic syntheses in general and is employed, besides for other uses, in the synthesis of the N-methyl-carbamate of 2,3-dihydro-2,2-dimethyl-benzofuran-7-ol of formula (IV):

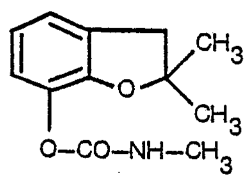

(IV)

by a multistage process.

Compound (IV) is known under the trade name "Carbofuran", and useful as active principle in different compositions of soil insecticides available on the market.

It is known from GB—A 2 006 211 to prepare compound (I) by selective mono-etherification of pyrocatechol (II) with methallyl halides (III), in the presence of inorganic bases in aprotic dipolar solvents, such as dimethylsulphoxide and N-methyl-2-pyrrolidone. These solvents, however, are expensive and not very stable, so that their recovery requires particular care and high operating efficiency. This adversely affects both operation and profitability. Moreover, the recovery of unreacted pyrocatechol from the resulting reaction mixtures presents some difficulties which entail high losses of the starting material along with high cost of operation in itself. It is therefore necessary to proceed with the highest possible conversions, which are only obtainable by employing the aforesaid sophisticated special solvents.

From GB—A 2 027 427 it is known to produce compound (I) by reacting pyrocatechol with methallyl chloride under an inert atmosphere in the presence of e.g. an alkalihydroxide or (bi) carbonate in a stirred two-phase liquid reaction medium, which comprises water, optionally a water inmiscible inert organic solvent and a quaternary ammonium or phosphonium compound as catalyst. This reaction is carried out under conditions of a so-called "phase transfer" reaction, which is certainly more complicated than usual reactions carried out in one single phase. Such phase transfer reactions have inherent operative difficulties and/or complications, e.g. in the separation of the reaction products and the recovery of the catalyst.

EPC application 80 104 521.2 describes the production of 7-hydroxycumaranes (including Carbofuran) by a two-step reaction, the first step of which is the production of the monomethallylether or pyrocatechol (I) by reacting pyrocatechol with a methallyl halogenide in the presence of a polyhydroxy alkylether with at least 1 hydroxy group as solvent. The use of other solvents is not described or mentioned in this prior application.

Nevertheless, operating under conditions directed to attain very high conversion values involves, in many cases, a practically unavoidable formation of by-products, such as dimethallyl-ether of pyrocatechol of formula (V):

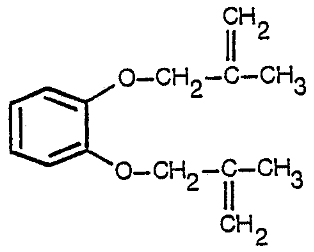

(V)

as well as of products alkylated on the aromatic ring. It is therefore absolutely necessary to reduce the formation of such by-products as much as possible in order not to jeopardize the economic and operative advantages expected from the high conversion.

It is therefore an object of the present invention to provide a method of preparing the mono-methallyl-ether of pyrocatechol free from the drawbacks of the prior art; in particular a process employing not only less expensive solvents than those proposed by the prior art, but also leading to high conversions and selectivities in the product to be obtained.

Thus, it has surprisingly been found that the use of particular hydroxylated solvents, homogeneously mixed with one another, as a reaction medium in the presence of organic or inorganic bases, leads to high conversions referred to pyrocatechol, maintaining acceptable selectivity values in the mono-methallyl-ether compound of formula (I), while the formation of by-products is maintained at admissible values.

The claimed process therefore overcomes the prejudice of the prior art, according to which an alkylation reaction of selective nature, like the reaction claimed herein, could not be carried out with hydroxylated solvents with high conversion and acceptable selectivity values.

The known prior art should have been an obstacle for the expert to carry out further researches in this direction when improved results were wanted.

The object of the invention is achieved by a process for selectively preparing the mono-methallyl-ether of pyrocatechol of formula (I), which is characterized in that pyrocatechol of formula (II) is reacted with a methallyl halide of formula (III), preferably the chloride, in a homogeneous solvent selected from saturated and unsaturated aliphatic alcohols with up to 5 carbon atoms, polyalcohols and the monoethers thereof;

water, and mixtures thereof (the sole use of said monoethers of polyalcohols being excluded from this invention), at temperatures from 30° to 120°C, in the presence of an organic or inorganic base. The reaction can be schematically represented by the following equation:

(1)

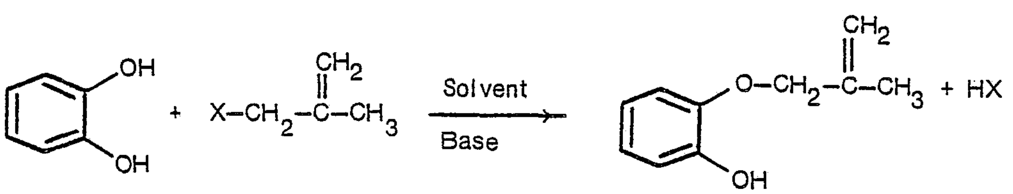


(II) (III) (I)

wherein X has the meaning already specified hereinbefore.

Preferred alcohols are linear and branched saturated and unsaturated aliphatic alcohols with up to 5 carbon atoms, in particular ethyl alcohol or allyl alcohol; polyalcohols, e.g. ethylene glycol, and monoethers thereof, e.g. methyl- and ethyl-cellosolve®. The use of aqueous mixtures of the above-said (poly)alcohols is of particular interest in economic and industrial respects.

Useful inorganic bases are oxides, hydroxides, carbonates, bicarbonates and acid phosphates of alkaline metals, preferably sodium and potassium carbonates and bicarbonates. Useful organic bases are e.g. tertiary amines, $N(R')_3$, in which $(R')_3$ is a homogeneous or heterogeneous hydrocarbyl group, such as triethylamine, and tetramethylguanidin.

The weight ratio between pyrocatechol (II) and the dissolving medium varies from 1:5 to 1:50, preferably from 1:10 to 1:30. The molar ratio between pyrocatechol (II) and the base varies from 1:0.5 to 1:3, preferably from 1:1 to 1:1.5.

The molar ratio between pyrocatechol (II) and methallyl halide (III) varies from 1:0.5 to 1:3, preferably from 1:1 to 1:1.5.

The reaction times range from 1 hour to 20 hours, preferably from 2 hours to 10 hours.

Suitable temperatures are from 30°C to 120°C, preferably of from 60°C to 90°C.

The pressure is substantially atmospheric pressure.

It is preferable to operate under a slight nitrogen or inert gas flow. The product (I) so obtained can be separated according to conventional techniques, such as extraction and distillation, after neutralization of the residual basicity in the reaction medium. Otherwise, the raw product of the reaction containing prevailingly product (I) can be utilized as such in the production of "Carbofuran", e.g. in a two-step process in which this mono-methallyl-ether is heated to 150°—200°C in a high-boiling neutral organic solvent and then cycled in the presence of acid catalysts.

The claimed process may be carried out as follows:

Pyrocatechol, the selected solvent, the selected organic or inorganic base and methallyl halide are introduced in any order into a thermoregulated reactor equipped with an agitator, a thermometer, a gas bubbler, a reflux cooler, and a feeding system for reagents.

Heating of the resulting reaction mixture begins under a continuous slight nitrogen flow until the desired reaction temperature is reached whereupon this temperature is maintained as long as necessary.

At the end of the reaction the resulting product can be separated according to known techniques, which will be described in more detail in the following examples. Subsequently the product — if necessary — may be analyzed according to conventional analytical techniques, such as gas-liquid chromatography.

As an alternative, the raw mono-methallyl-ether of pyrocatechol can be directly subjected to treatments known from the art.

The new process is particularly advantageous due to its mild and simple operative conditions and to the selectivity of the obtained product.

The claimed process is described in more details in the following examples, which are given only for illustrative purposes.

Example 11 shows the production of "Carbofuran".

## Example 1

12 g of sodium carbonate, 11 g of pyrocatechol, 100 ml of 95% ethanol and 20 ml of methallyl chloride were introduced into a thermoregulated 250-ml flask, equipped with magnetic stirrer, reflux cooler, thermometer, gas bubbler and a feeding system for reagents. Under a slight nitrogen flow the system was heated at reflux (about 70°C) and maintained at such temperature for about 5 hours.

At the conclusion of the reaction it was acidified with $H_2SO_4$ at 10% and was extracted with three portions of 100 ml each of ethyl ether.

The ethereal extract, after dilution to the desired volume, was subjected to gas-chromatographic analysis. Such analysis revealed the presence of 11.6 g of (I), corresponding to a yield of 71%, referring to the starting pyrocatechol.

The monoether (I)/diether(IV) molar ratio was 3.6:1.

## Example 2

Utilizing the apparatus described in example 1, 11 g of pyrocatechol, 16.8 g of sodium bicarbonate, 80 ml of anhydrous ethanol and 20 ml of methallyl chloride were introduced into the reactor.

After 7 hours at about 70°C, the gas-chromatographic analysis of the reaction mixture, separated as in example 1, revealed that 11.5 g of monoether (I) had formed, with a yield of 70.3%, referring to the starting pyrocatechol, and a monoether/diether molar ratio of 9.7:1.

## Examples 3—10

By utilizing the apparatus described in example 1 and varying the operative conditions, solvent and basic system, the results recorded in the following Table were obtained. The mono- and diether yields are molar yields calculated on the starting pyrocatechol. Analogously the mono-/diether ratio was calculated by moles. In all the examples, 0.1 mole of pyrocatechol in 80 ml of solvent were employed. TMG means tetramethylguanidin.

| Example | Solvent | Base [moles] | Metallyl chloride [moles] | Temperature [°C] | Time [h] | Mono [%] | DI [%] | Mono/DI ratio |
|---|---|---|---|---|---|---|---|---|
| 3 | Anhydrous ethanol | $Na_2CO_3$ [0.1] | 20 ml [0.2] | 75 | 7 | 54 | 3 | 18 |
| 4 | Anhydrous ethanol | TMG [0.12] | 20 ml [0.2] | 75 | 4 | 64.7 | 20.8 | 3.1 |
| 5 | Methanol | $NaHCO_3$ [0.2] | 20 ml [0.2] | 65 | 7 | 54.6 | 28.8 | 1.9 |
| 6 | $H_2O$ | $NaHCO_3$ [0.2] | 20 ml [0.2] | 55 | 7 | 21 | 1.2 | 17.5 |
| 7 | $H_2O$/Ethanol 1:1 | $Na_2CO_3$ [0.11] | 15 ml [0.15] | 70 | 2 | 54.9 | 8 | 6.8 |
| 8 | $H_2O$/Methanol 1:1 | $Na_2CO_3$ [0.11] | 15 ml [0.15] | 65 | 2 | 45.4 | 5.8 | 7.8 |
| 9 | Ethanol at 95% | $NaHCO_3$ [0.2] | 20 ml [0.2] | 70 | 7 | 65.8 | 19.5 | 3.4 |
| 10 | Anhydrous ethanol | NaOH [0.11] | 20 ml [0.2] | 75 | 2 | 55.3 | 16.0 | 3.3 |

Example 11

106 g of sodium carbonate, 110 g of pyrocatechol, 1000 ml of 95% ethanol and 200 ml of methallyl chloride were introduced into a thermoregulated 2000 ml reactor, equipped with mechanical stirrer, reflux cooler, thermometer, gas bubbler and a feeding system for reagents.

The system was heated at reflux (about 70°C) and kept at such temperature for about 10 hours.

At the conclusion of the reaction 300 ml of ortho-dichlorobenzene were added and the excess of methallyl chloride and ethanol at 95% were distilled off; 879 g of distillate containing about 66 ml of methallyl chloride were collected.

After cooling, 500 ml of 10% $H_2SO_4$ were gradually introduced into the reactor.

The two phases were separated and the aqueous phase was again extracted with 200 ml of ortho-dichloro-benzene.

The organic solution of ortho-dichloro-benzene was transferred into a 1000 ml reactor and heated at reflux (about 180°C) under a nitrogen flow.

It was maintained at such temperature for 2 hours, then cooled to 40°C, and 2 g of p-toluenesulphonic acid were charged. The mixture was heated to 60°C for further 2 hours, under a nitrogen flow.

Successively, 1 g of sodium bicarbonate was introduced and stirring was continued for further 30 minutes.

There were distilled first ortho-dichloro-benzene, which passed at 60—70°C at about 13.3 mbar, then the desired product which passed at to 90°—125°C at 1.06 mbar.

The two united fractions were extracted under a nitrogen flow with three portions of 5% NaOH of 33 ml each: the aqueous phase was then acidified with 10% $H_2SO_4$ and again extracted with three portions of 300 ml each of methylene chloride.

The organic extract after drying on sodium sulphate was transferred into a 1000 ml flask and treated with 30 ml of methylisocyanate and 1 ml of triethylamine.

The whole was maintained at 30°C for about 1 hour, whereupon solvent was evaporated under a slight vacuum, 94 g of raw Carbofuran were obtained.

Titre = 85.4%. Yield referred to the starting pyrocatechol = 36.3%.

## Claims

1. A process for preparing the mono-methallyl-ether of pyrocatechol of formula (I):

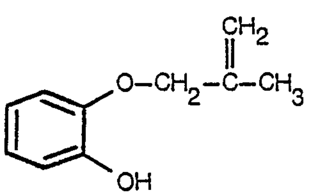

(I)

characterized in that pyrocatechol is reacted with a methally halide in a homogeneous hydroxylated dissolving medium selected from saturated and unsaturated aliphatic alcohols with up to 5 carbon atoms in the molecule, polyalcohols and monoethers thereof;
water and mixtures thereof, the sole use of the said monoethers of polyalcohol being excluded, in the presence of an organic or inorganic base at temperatures from 30° to 120°C.

2. A process according to claim 1, characterized in that the methallyl halide is methallyl chloride.

3. A process according to any one of the preceding claims, characterized in that the solvent is selected from ethyl alcohol, ethylene glycol and allyl alcohol and mixtures thereof.

4. A process according to any of the preceding claims, characterized in that the inorganic base is selected from oxides, hydroxides, carbonates, bicarbonates and acid phosphates of alkaline metals.

5. A process according to claim 4, characterized in that the inorganic base is selected from sodium and potassium carbonates and bicarbonates.

6. A process according to any one of the preceding claims 1—3, characterized in that the organic base is selected from tertiary amines of formula $N(R')_3$ in which $(R')_3$ is a homogeneous or heterogeneous hydrocarbyl group, preferably triethylamine and tetramethylguanidin.

7. A process according to any one of the preceding claims, characterized in that the weight ratio between the pyrocatechol and the dissolving medium ranges from 1:5 to 1:50 and preferably from 1:10 to 1:30.

8. A process according to any one of the preceding claims, characterized in that the molar ratio between the pyrocatechol and the organic or inorganic base ranges from 1:0.5 to 1:3, preferably from 1:1 to 1:1.5.

9. A process according to any one of the preceding claims, characterized in that the molar ratio between the pyrocatechol and the methallyl halide ranges from 1:0.5 to 1:3, preferably from 1:1 to 1:1.5.

10. A process according to any one of the preceding claims, characterized in that the temperature ranges from 60° to 90°C.

11. A process according to any one of the preceding claims, characterized in that it is conducted in an inert atmosphere, preferably in a nitrogen atmosphere.

**Patentansprüche**

1. Verfahren zur Herstellung des Monomethallylethers von Brenzkatechin der Formel (I)

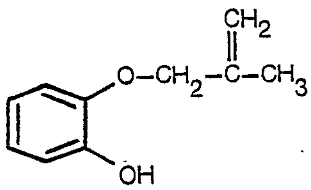

(I)

dadurch gekennzeichnet, daß man Brenzkatechin mit einem Methallylhalogenid in einem homogenen hydroxylieten lösenden Medium, ausgewählt aus gesättigten und ungesättigten aliphatichen Alkoholen mit bis zu 5 Kohlenstoffatomen im Molekül, Polyalkoholen und Monoethern derselben; Wasser und Mischungen derselben, wobei die alleinige Verwendung dieser Monoether von Polyalkohol ausgeschlossen ist, in Anwesenheit einer organischen oder anorganischen Base bei Temperaturen von 30 bis 120°C umsetzt.

2. Verfahren nach Anspruch 1, dadurch gekennzeichnet, daß das Methallylhalogenid Methallylchlorid ist.

3. Verfahren nach einem der vorhergehenden Ansprüche, dadurch gekennzeichnet, daß das Lösungsmittel ausgewählt ist aus Ethylalkohol, Ethylenglykol und Allylalkohol sowie Mi-schungen derselben.

4. Verfahren nach einem der vorhergehenden Ansprüche, dadurch gekennzeichnet, daß die anorganische Base ausgewählt ist aus Oxiden, Hydroxiden, Carbonaten, Bicarbonaten und sauren Phosphaten von Alkalimetallen.

5. Verfahren nach Anspruch 4, dadurch gekennzeichnet, daß die anorganische Base ausgewählt ist aus Natrium- und Kalium-carbonaten und -bicarbonaten.

6. Verfahren nach einem der vorhergehenden Ansprüche 1 bis 3, dadurch gekennzeichnet, daß die organische Base ausgewählt ist aus tertiären Aminem der Formel $N(R')_3$, in welcher $(R')_3$ eine homogene oder heterogene Hydrocarbylgruppe ist, vorzugsweise Triethylamin und Tetramethylguandin.

7. Verfahren nach einem der vorhergehenden Ansprüche, dadurch gekennzeichnet, daß das Gewichtsverhältnis zwischen Brenzkatechin und dem lösenden Medium im Bereich von 1:5 bis 1:50, vorzugsweise von 1:10 bis 1:30, liegt.

8. Verfahren nach einem der vorhergehenden Ansprüche, dadurch gekennzeichnet, daß das molare Verhältnis zwischen Brenzkatechin und der organischen oder anorganischen Base im Bereich von 1:0,5 bis 1:3, vorzugsweise von 1:1 bis 1:1,5, liegt.

9. Verfahren nach einem der vorhergehenden Ansprüche, dadurch gekennzeichnet, daß das molare Verhältnis zwischen Brenzkatechin und dem Methallylhalogenid im Bereich von 1:0,5 bis 1:3, vorzugsweise von 1:1 bis 1:1,5, liegt.

10. Verfahren nach einem der vorhergehenden Ansprüche, dadurch gekennzeichnet, daß die Temperatur im Bereich von 60 bis 90°C liegt.

11. Verfahren nach einem der vorhergehenden Ansprüche, dadurch gekennzeichnet, daß es in einer inerten Atmosphäre, vorzugsweise in einer Stickstoffatmosphäre, durchgeführt wird.

**Revendications**

1. Un procédé de préparation de monométhallyléther de pyrocatéchol de formule (I):

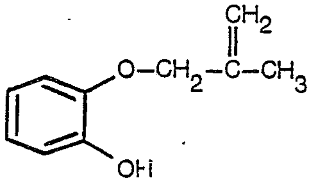

(I)

caractérisé en ce que le pyrocatéchol réagi avec un halogénure de méthallyle dans un milieu de dissolvant hydroxylé homogène choisi parmi les alcools aliphatiques saturés et insaturés comprenant jusqu'à 5 atomes de carbone dans la molécule, les polyalcools et leurs monoéthers, l'eau et ses

mélanges, l'emploi de monoéthers de polyalcools seuls étant prescrit, en présence d'une base organique ou minérale à des températures comprises entre 30° et 120°C.

2. Un procédé selon la revendication 1, caractérisé en ce que l'halogénure de méthallyle est le chlorure de méthallyle.

3. Un procédé selon l'une quelconque des revendications précédentes, caractérisé en ce que le solvant est choisi parmi l'alcool éthylique, l'éthylène glycol et l'alcool allylique et leurs mélanges.

4. Un procédé selon une quelconque des revendications précédentes, caractérisé en ce que la base organique est choisie parmi les oxydes, hydroxydes, carbonates, bicarbonates et phosphates acides de métaux alcalins.

5. Un procédé selon la revendication 4, caractérisé en ce que la base minérale est choisie parmi les carbonates et bicarbonates de sodium et de potassium.

6. Un procédé selon une quelconque des revendications 1 à 3 précédentes, caractérisé en ce que la base organique est choisie parmi les amines tertiaires de formule $N(R')_3$ dans laquelle $(R')_3$ est un groupe hydrocarboné homogène ou hétérogène, de préférence la triéthylamine et la tétraméthylguanidine.

7. Un procédé selon une quelconque des revendications précédentes, caractérisé en ce que le rapport pondéral pyrocathéchol/milieu dissolvant est compris entre 1/5 et 1/50, et de préférence entre 1/10 et 1/30.

8. Un procédé selon l'une quelconque des revendications précédentes, caractérisé en ce que le rapport molaire pyrocatéchol/base organique ou minérale est compris entre 1/0,5 et 1/3, et de préférence 1/1 et 1/1,5.

9. Un procédé selon une quelconque des revendications précédentes, caractérisé en ce que le rapport molaire entre le pyrocatéchol et l'halogénure de méthallyle est compris entre 1/0,5 et 1/3, et de préférence entre 1/1 et 1/1,5

10. Un procédé selon une quelconque des revendications précédentes, caractérisé en ce que la température est comprise entre 60° et 90°C.

11. Un procédé selon une quelconque des revendications précédentes, caractérisé en ce qu'il est mis en oeuvre dans une atmosphère inerte, de préférence dans une atmosphère d'azote.